# EUROPÄISCHE PATENTSCHRIFT

(11) **EP 0 769 141 B1**
(45) Veröffentlichungstag und Bekanntmachung des Hinweises auf die Patenterteilung: **16.12.1998**
(21) Anmeldenummer: 95919446.5
(22) Anmeldetag: 12.05.1995
(51) Int. Cl.: G01N 27/12

(54) **GASSENSOR FÜR REDUZIERENDE ODER OXIDIERENDE GASE**
SENSOR FOR REDUCING OR OXIDIZING GASES
DETECTEUR POUR GAZ REDUCTEURS OU OXYDANTS

(30) Priorität: 02.07.1994 DE 4423289
(43) Veröffentlichungstag der Anmeldung: 23.04.1997
(73) Patentinhaber: Forschungszentrum Karlsruhe GmbH, 76133 Karlsruhe (DE)
(72) Erfinder: ALTHAINZ, Peter, D-76135 Karlsruhe (DE); GOSCHNICK, Joachim, D-75417 Mühlacker (DE)
(74) Vertreter: Rückert, Friedrich, Dr.
(86) Internationale Anmeldenummer: EP9501799
(87) Internationale Veröffentlichungsnummer: WO9601419

(56) Entgegenhaltungen:
- EP-A- 0 364 315
- GB-A- 2 170 913
- US-A- 4 457 161
- SENSORS AND ACTUATORS, Bd.13-14, Seiten 458 - 461 XIADONG WANG ET AL. 'An integrated array of multiple thin film metal oxide sensors for quatification of individual components in organic vapor mixtures' in der Anmeldung erwähnt
- SENSORS AND ACTUATORS, Bd.12, Seiten 103 - 110 M. S. NAYAK ET AL. 'Transformed cluster analysis: an approach to the identification of gases/odours using an integrated gas-sensor array.' in der Anmeldung erwähnt

## Beschreibung

Die Erfindung betrifft einen Gassensor für reduzierende oder oxidierende Gase gemäß dem Oberbegriff von Anspruch 1.

Ein solcher Gassensor ist aus der Veröffentlichung von X. Wang, S. Yee und P. Carey, Sensors and Actuators B, 13-14 (1993) 458-461 bekannt. Dieser Gassensor besteht aus einer Anordnung von acht Einzelsensoren, die paarweise auf einem Silicium-Chip angeordnet sind. Jeder dieser acht Einzelsensoren besteht aus einem oder mehreren der halbleitenden Oxide SnO₂, ZnO, TiO₂ und WO₃ und ist als Dünnfilm auf dem Chip aufgetragen. Teilweise sind die Einzelsensoren mit Palladium als Katalysator überdeckt. Alle Einzelsensoren unterscheiden sich in ihrer Zusammensetzung oder ihrem Aufbau. In Kontakt mit Meßgasen verändert sich die Leitfähigkeit der halbleitenden Oxide in Abhängigkeit von ihrer Zusammensetzung und ggf. der darauf angebrachten katalytisch wirkenden Beschichtung. Somit liefert jeder Einzelsensor in Kontakt mit einem Meßgas ein unterschiedliches Signal, das der Änderung der Leitfähigkeit proportional ist. Die Leitfähigkeitsänderungen aller Einzelsensoren werden gemessen und - nach Eichung des Gassensors mit Hilfe mathematischer Methoden - der Konzentration eines oder mehrerer Bestandteile des Meßgas zugeordnet. Die Meßtemperatur beträgt 200°C. Es ist angegeben, daß die dampf- bzw. gasförmigen Komponenten Methanol, Aceton, Benzol, Toluol, TCE und CO in einem Meßgas analytisch bestimmt werden können.

Die Herstellung eines solchen Gassensors erscheint schwierig, da alle Einzelsensoren separat hergestellt und jeweils mit zwei Elektroden versehen werden müssen.

Ein ähnlicher Gassensor ist aus der Veröffentlichung von M. S. Nayak, R. Dwivedi und S. K. Srivastava in Sensors and Actuators B, 12 (1993) 103 - 110 bekannt. In dieser Veröffentlichung finden sich die theoretischen Grundlagen zur Auswertung eines aus Einzelsensoren bestehenden Gassensors.

In der Veröffentlichung von H. V. Shurmer, J. W. Gardner und P. Corcoran, Sensors and Actuators B1, (1990) 256 - 260 ist ein Gassensor mit zwölf Zinnoxid-Einzelsensoren beschrieben. Die Einzelsensoren unterscheiden sich in ihrer Charakteristik. Die Veröffentlichung beschreibt die elektrische Verschaltung der Einzelsensoren, so daß ein Gasgemisch analytisch bestimmt werden kann.

Aus der Veröffentlichung von P.Althainz, A.Dahlke, M.Frietsch-Klarkopf, J.Goschnick und H.J.Ache, Physica Status Solidi (a) 145, 611 (1994) ist ein Gassensor für organische Gase bekannt, der auf einem Siliciumdioxid-Substrat eine gasempfindliche Schicht aus Zinndioxid trägt. Auf der gasempfindlichen Schicht sind Goldelektroden aufgetragen, mit deren Hilfe die Leitfähigkeitsänderungen der gasempfindlichen Schicht in Kontakt mit einem Meßgas erfaßt werden können. Die Herstellung des Sensors mit Hilfe der Dünnschichttechnik wird eingehend beschrieben.

Aus der DE 36 10 363 A1 ist ein Verfahren zum kontinuierlichen Überwachen von Konzentrationen von gasförmigen Bestandteilen in Gasgemischen, ausgenommen Sauerstoff, bekannt. Dieses Verfahren verwendet elektrochemische Zellen, deren Elektroden aus Metalloxiden bestehen. Die Elektroden können teilweise von einer katalytischen Schicht abgedeckt sein oder eine unterschiedliche Zusammensetzung aufweisen. In diesem Fall müssen die Elektroden jedoch durch ein inertes Material voneinander abgetrennt werden.

Aufgabe der Erfindung ist, einen Gassensor der eingangs genannten Art vorzuschlagen, dessen Herstellung einfacher ist. Mit diesem Sensor sollen sich reduzierende Gase wie z. B. Wasserstoff, Methan, Kohlenmonoxid, Ethanol im Dampfzustand etc. und oxidierende Gase wie z. B. Stickstoffdioxid analytisch bestimmen lassen, d. h. in einem Gemisch mehrerer Komponenten sollen die Einzelkomponenten identifiziert und quantifiziert werden.

Die Aufgabe wird erfindungsgemäß durch einen Gassensor mit den gekennzeichneten Merkmalen gelöst. Die abhängigen Ansprüche beschreiben bevorzugte Ausgestaltungen des erfindungsgemäßen Gassensors.

Mit der Bezeichnung "reduzierende oder oxidierende Gase" soll klargestellt werden, daß sich mit dem erfindungsgemäßen Sensor prinzipiell alle Gase außer den Inertgasen wie Stickstoff, Kohlendioxid und Edelgase analytisch bestimmen lassen.

Die gegenüber dem Meßgas bzw. seinen Komponenten empfindliche Schicht des erfindungsgemäßen Gassensors besteht im Gegensatz zum eingangs beschriebenen Gassensor aus einer einzigen, durchgehenden Schicht aus einem oder mehreren halbleitenden Oxiden wie z. B. Zinndioxid. Die durchgehende Schicht wird durch bandförmige Elektroden in einzelne Felder unterteilt. Die äußeren Elektroden definieren die Gesamtfläche der durchgehenden Schicht, während durch die inneren Elektroden einzelne Felder der durchgehenden Schicht voneinander getrennt werden. Im Gegensatz zum eingangs beschriebenen Sensor, der 2n Elektroden benötigt, sind nur (n+1) Elektroden notwendig, wenn n die Zahl der Felder angibt. Die Elektroden können direkt auf oder unter der Oberfläche der durchgehenden Schicht aufgetragen werden. Vorzugsweise werden bandförmige Elektroden eingesetzt, die zueinander parallel verlaufen und jeweils über die gesamte Breite der durchgehenden Schicht geführt sind. Die Elektroden bilden hierbei ein paralleles Streifenmuster und trennen die einzelnen Felder voneinander ab. Über jeweils zwei dieser Elektroden kann die Änderung der elektrischen Leitfähigkeit, die durch die Art und Konzentration eines Meßgases beeinflußt wird, bestimmt werden, wobei die Leitfähigkeitsänderung derjenigen Felder gemessen wird, die von den erfaßten Elektroden eingeschlossen werden. Im Gegensatz zum eingangs beschriebenen Gassensor bestehen daher wesentlich mehr Schaltungsmöglichkeiten, die für die Analyse ausgenützt werden können.

Zum Beispiel können sowohl Widerstandsmessungen zwischen zwei benachbarten Elektroden, als auch Messungen zwischen vier Elektroden durchgeführt werden. Die hohe Anzahl an Feldern mit jeweils nur geringfügigen Unterschieden von einem Feld zum nächsten beinhaltet eine Redundanz der Daten, sodaß fehlerhafte Elemente durch stark abweichendes Verhalten erkannt und selbsttätig ausgeschaltet werden können. Letztendlich ist es auch möglich integral über mehrere bzw. alle Felder zu messen, indem nur die äußeren Elektroden genutzt werden. Unter Verlust der erkennenden Eigenschaften kann damit eine gesteigerte Empfindlichkeit erzielt werden.

Eine weitere Einsatzmöglichkeit der Elektroden betrifft die weiter unten erwähnte Heizung des Sensors. Von den vielen Einzelelektroden können mehrere (in einem Ausführungsbeispiel sind das die beiden äußeren Elektroden) mit Verbindungsflächen zur elektrischen Kontaktierung an beiden Seiten der Elektrode versehen werden, sodaß nicht nur der Widerstand des Feldes, sondern auch der des Platin Kontaktbandes an sich gemessen werden kann. Dieser dient zur genauen Temperaturbestimmung und -regelung (Platin-Widerstandsthermometer).

Damit mehrere Komponenten eines Meßgases analytisch bestimmt werden können, müssen sich die einzelnen Felder in ihrem Aufbau oder in ihrer Zusammensetzung voneinander unterscheiden, so daß für jedes Feld eine gegenüber den anderen Feldern unterschiedliche Leitfähigkeitsänderung erhalten wird, wenn der Sensor mit einem einzigen Gas in Kontakt gebracht wird. Die unterschiedliche Leitfähigkeitsänderung kann einerseits durch eine unterschiedliche Zusammensetzung oder Dotierung der halbleitenden Metalloxide erreicht werden.

In diesem Fall besteht die Möglichkeit, für jedes einzelne Feld eine in sich homogene Zusammensetzung vorzusehen, die sich jedoch von den übrigen Feldern unterscheidet. Eine solche Anordnung kann hergestellt werden, indem der Metalloxidfilm durch Aufdampfen von Edelmetallen dotiert wird und während des Aufdampfvorgangs eine planare Blende seitlich schrittweise über die Felder geschoben wird, so daß jedes Feld eine unterschiedliche Zeitdauer bedampft wird und damit eine stufenweise Konzentrationsänderung der Dotierung von Feld zu Feld erreicht wird.

Eine Alternative besteht darin, daß sich die Zusammensetzung über die durchgehende Schicht hinweg kontinuierlich ändert. Eine Herstellungsmöglichkeit hierfür bietet die Gasphasenabscheidung (Chemical Vapor Deposition, CVD), wenn beispielsweise zwei Quellen vorgesehen werden, von denen eine über dem ersten Ende und die andere über dem zweiten Ende des für die durchgehende Schicht vorgesehenen Bereichs angeordnet werden. Die Gasphasenabscheidung gestattet außerdem, die chemische Zusammensetzung moleküllagenweise zu kontrollieren. Hierzu werden Vorläufersubstanzen bei niedrigen Drücken verdampft und erst auf einem Substrat durch thermische oder andersartige Energiezufuhr zu der gewünschen Verbindung umgesetzt. Bei diesen Verfahren ergibt sich eine Zusammensetzung des Dünnfilms, die sich vom ersten Ende der durchgehenden Fläche zu deren zweitem Ende kontinuierlich ändert, wobei die Enden der Zusammensetzung der über ihnen angeordneten Quellen entspricht.

Die unterschiedliche Leitfähigkeitsänderung kann andererseits durch eine unterschiedliche Beschichtung der durchgehenden Schicht erreicht werden, wobei die durchgehende Schicht selbst in ihrer Zusammensetzung einheitlich sein kann. Zur Beschichtung sind z. B. die Oxide Al₂O₃ oder SiO₂ geeignet. Durch die Beschichtung kann die Durchlässigkeit der Komponenten eines Meßgases beeinflußt werden, so daß der Kontakt der einzelnen Komponenten eines Gasgemisches mit einzelnen Feldern des halbleitenden Metalloxid-Dünnfilms je nach ihrer Molekülstruktur unterschiedlich stark behindert wird.

Die Zusammensetzung oder die Dicke der Beschichtung kann sich zwischen den äußeren Elektroden stetig ändern. Die Änderung der Zusammensetzung kann durch die oben beschriebene Verfahren der Gasphasenabscheidung erreicht werden. Im einfachsten Fall ist über der durchgehenden Schicht mit einer homogenen Zusammensetzung eine Beschichtung aufgebracht, deren Dicke vom Ort der ersten äußeren Elektrode zum Ort der zweiten äußeren Elektrode stetig ansteigt, so daß der Querschnitt der Beschichtung eine keilförmige Gestalt aufweist.

Der Gassensor muß bei einer Temperatur von einigen hundert °C, z. B. zwischen 300 und 400° C betrieben werden. Er wird daher vorzugsweise mit einer integrierten Heizung versehen. Ist die durchgehende Fläche auf einem plattenförmigen Substrat, etwa einem Silicium-Chip, aufgebracht, kann die freie Seite des Substrats mit einer mäanderförmigen Heizleiterbahn versehen werden. Die Heizleiterbahn läßt sich durch Aufstäuben (Sputtern) eines geeigneten Metalls, z. B. Platin, herstellen.

Da die Temperatur der durchgehenden Schicht die Leitfähigkeitsänderungen beeinflußt, kann der Temperatureffekt zur Veränderung der Leitfähigkeit herangezogen werden. Es können mehrere verschiedene Heizleiterbahnen auf der freien Substratseite angebracht werden, deren Fläche einem oder mehreren Feldern entspricht und die diesen Feldern zugeordnet werden. Auf diese Weise können einzelne Felder oder Gruppen von Feldern auf einer gegenüber den Nachbarfeldern unterschiedlichen Temperatur gehalten werden.

Schließlich kann eine chemisch wirksame Schicht vorgesehen werden, die das zu messende Gas chemisch umsetzt, so daß nicht das Gas selbst, sondern sein Reaktionsprodukt analytisch erfaßt wird. Das Reaktionsprodukt muß dann ein reduzierendes oder oxidierendes Gas sein.

In den Figuren sind eine Ausführungsform des erfindungsgemäßen Gassensors und Masken zu ihrer Herstellung dargestellt. Es zeigen:
Fig. 1 die Ausführungsform von der Unterseite (a) und der Oberseite (b) und (c) und im Querschnitt (d),
Fig. 2 den Aufbau der Ausführungsform,
Fig. 3 Masken zur Herstellung der Ausführungsform.
Fig. 4 eine Ausführungsform mit Gehäuse (a) und Teilansichten der Heizung (b), der Heizungskontaktierung (c), der Elektroden (d), (e) sowie des Gehäuses (f).

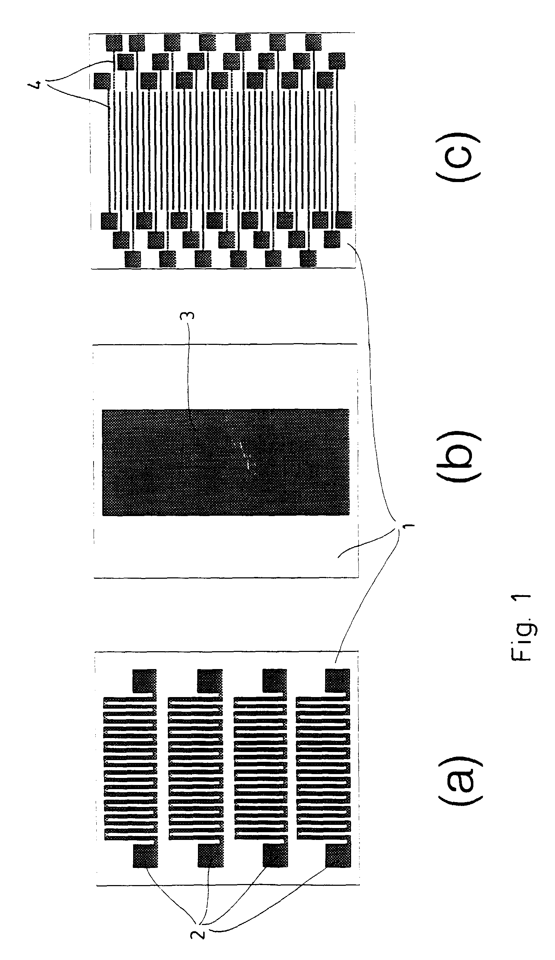

Der in Fig. 1 dargestellte Sensor ist auf einem plattenförmigen Substrat 1 in Form eines Silicium-Wafers aufgebaut. Teil (a) zeigt die Unterseite des Substrats. Auf die Unterseite des Substrats 1 sind vier Heizelemente 2 aus Platin durch Aufstäuben (Sputtern) aufgetragen, mit deren Hilfe Gruppen von Feldern auf eine unterschiedliche Temperatur gebracht werden können. Die Teile (b) und (c) der Figur zeigen die Oberseite des Substrats 1. In Teil (b) ist die durchgehende Schicht 3 aus SnO₂ dargestellt. Teil (c) zeigt die streifenförmigen Elektroden 4, die sich über die gesamte Breite der durchgehenden Schicht 3 erstrecken. Durch die Elektroden 4 werden aus der durchgehenden Schicht 3 streifenförmige Felder voneinander getrennt.

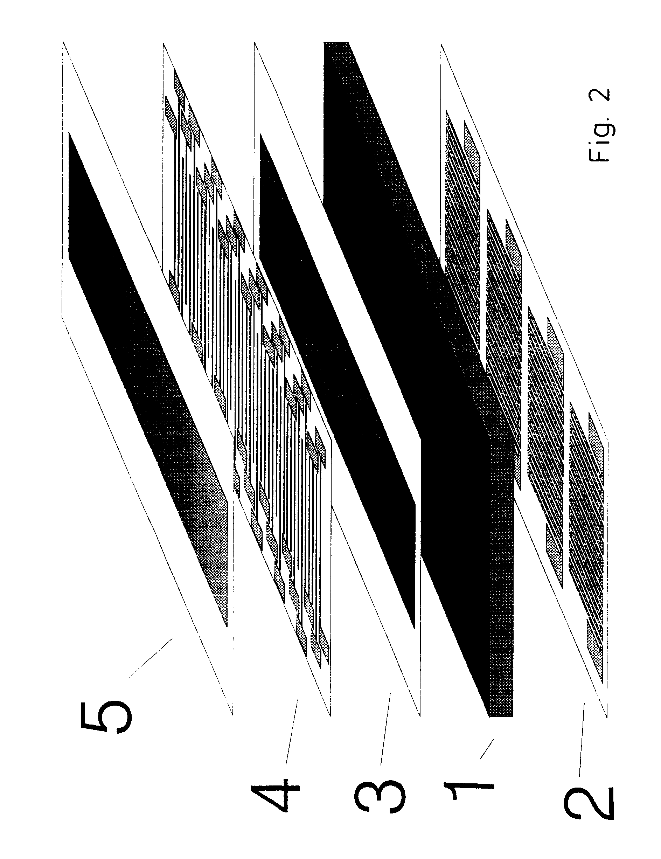

Der Sensor nach Fig. 2 ist mit einer zusätzlichen Beschichtung 5 versehen. Die Dicke der Beschichtung ändert sich stetig zwischen den beiden äußeren Elektroden.

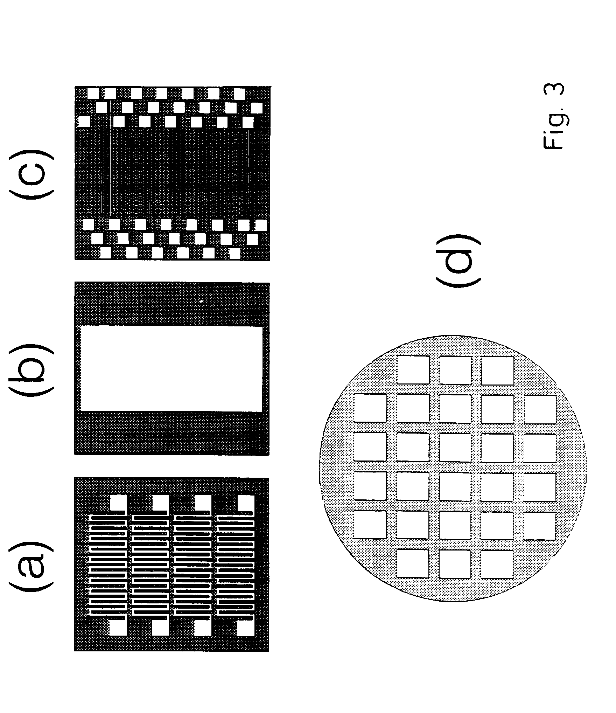

In Fig. 3 sind Masken dargestellt, mit deren Hilfe der in Fig. 2 dargestellte Sensor hergestellt werden kann. Alle Bestandteile des Sensors werden mit Hilfe dieser Masken in Schattenmaskentechnik hergestellt. Die Masken können beispielsweise aus rechteckigen, 0,5 mm dicken Nickelblechen mit den Methoden der Mikrostrukturtechnik hergestellt werden.

Teil (a) zeigt die Maske, die für die Herstellung einer vierfach unterteilten Heizung eingesetzt wird.

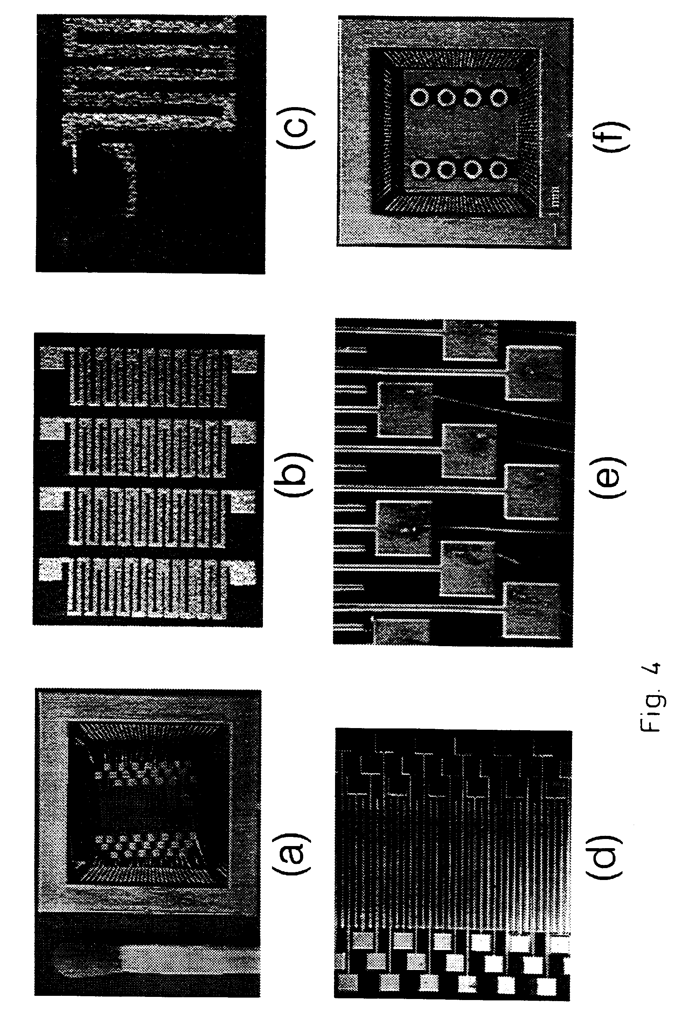

Fig. 4 zeigt Ansichten eines fertiggestellten Gassensors,

Die Erfindung wird nachfolgend anhand von Durchführungsbeispielen näher erläutert.

### Beispiel 1:

### Herstellung der Metalloxidschicht, der Elektroden und der Heizung eines Gassensors

Als Ausgangsmaterial wurden Silizium-Scheiben mit drei Zoll Durchmesser und einer Dicke von 0,5 mm verwendet. Auf beiden Seiten der Scheiben befindet sich eine 500 nm SiO₂-Schicht zur elektrischen Isolierung. Alle Schichten, mit Ausnahme der lateral stetig sich ändernden Beschichtung über den Feldern, wurden mit der Hochfrequenzionenzerstäubungstechnik aufgebracht (Magnetron-Sputtern). Die Mikrostrukturierung erfolgt durch metallische Schattenmasken der Dicke 0,05 mm, die Ausparungen an den Stellen aufweisen, an denen Material aufgebracht werden soll. Die Masken werden auf dem Substrat fixiert und beides wird in die Magnetron Anlage eingebracht. Insgesamt finden auf jeder Scheibe 26 einzelne Sensoren der Abmessung 8 x 9 mm Platz. Jeder dieser Sensoren verfügt über 40 bänderförmige Elektroden und damit 39 Felder. Jede Elektrode ist 50 µm breit und der Abstand zwischen den Elektroden beträgt 150 µm.

Zuerst wird auf der Rückseite der Scheibe in 1000 nm Dicke eine Mäanderheizung aus Platin aufgesputtert. Als Haftvermittler dient eine 30 nm dicke Ti-Schicht. Auf die Bondflächen wird zum Schluß eine mehrere µm dicke Ag-Schicht gesputtert.

Nach Herstellung der Heizung wird das Substrat gewendet und die Maske für den Metalloxidfilm montiert. SnO₂ in 150 nm Dicke wird durch reaktives Magnetron Sputtern mit einer Argon-Sauerstoff-Mischung von 80/20 und einer Leistung von 60 Watt hergestellt.

Danach wird die Maske für die Elektrodenstruktur montiert. Zunächst werden die SnO₂-Felder abgedeckt und eine Ti-Haftvermittlerschicht für die Haftung der Bondflächen auf der Scheibe hergestellt. Nach Entfernen dieser Abdeckung werden die eigentlichen Platin-Kontakte in einer Dicke von 1000 nm gesputtert. Am Ende steht wieder die Abscheidung eine 1000 nm dicken Ag-Schicht auf den Bondflächen, um die Kontaktierung eines Drahtes zu gewährleisten.

Im letzten Schritt wird die Scheibe in die 26 vorhandenen Einzelchips zersägt. Die einzelnen Sensorchips werden in ein kommerziell erhältliches IC-Gehäuse eingebaut und die elektrischen Verbindungen zum Chip durch Ultraschall-Verbindung mit Aluminiumdraht (Elektroden) und Golddraht (Heizung) hergestellt.

### Beispiel 2:

### Herstellung eines Gassensors mit im Querschnitt keilförmiger Beschichtung.

SiO₂ wird nach dem Verfahren der Ionenstrahlunterstützten Gasphasenabscheidung (IBAD, Ion Beam Assisted Deposition) hergestellt. Dieses Verfahren ist in einer Veröffentlichung [P. Althainz, A. Dahlke, M. Frietsch-Klarhof, J. Goschnick and H. J. Ache, "Organically modified SiO₂- and Al₂O₃-Films as Selective Components for Gassensors", Physica Status Solidi, to be published] beschrieben. Dabei wird gasförmiges Tetraethoxysilan (TEOS) durch Ionenstrahlanregung zu SiO₂ umgesetzt. Die Ionenstrahlanregung läßt sich durch eine Blende auf einen schmalen Bereich begrenzen, so daß nur auf diesem Bereich SiO₂ abgeschieden wird. Auf diese Weise wurde mit einer 2 mm breiten Spaltblende durch kontinuierliches Bewegen eines Substrats unter der Blende während der Bedampfung eine Membran mit einem Dickegradienten erzeugt. Die Schicht hatte laterale Abmessungen von 20 x 20 mm² und wies auf der einen Seite eine Dicke von 6 nm und auf der anderen Seite eine Dicke von 8 nm auf.

### Beispiel 3:

### Herstellung eines Sensors mit sich stetig ändernder Zusammensetzung des halbleitenden Metalloxid-Dünnfilms.

Die Herstellung eines Sensors mit sich stetig ändernder Zusammensetzung des halbleitenden Metalloxid-Dünnfilms ist möglich, indem eine Anordnung mit zwei Magnetron-Sputterquellen gewählt wird und mit der einen Quelle SnO₂ erzeugt wird, während die zweite Quelle z.B. ZnO sputtert. Eine stetig variierende Zusammensetzung entsteht, indem beide Quellen nicht parallel gegenüber dem Substrat, sondern seitlich vom Substrat angebracht werden und damit bewußt eine inhomogene Beschichtung erzeugt wird und zwar in der Weise, daß die eine Quelle dort mehr Material deponiert, wo die zweite Quelle weniger abscheidet.

### Beispiel 4:

### Herstellung eines Sensors mit sich stetig ändernder Zusammensetzung der den Metalloxid-Dünnfilm überdeckenden Beschichtung.

Mit ionenstrahlunterstützter Deposition können auch andere Oxidschichten hergestellt werden. Eine Veröffentlichung [D. Leinen, A. Fernández, J. P. Espinós, T. R. Belderrain and A. R. González-Elipe,"Ion beam induced chemical vapor deposition for the preparation of thin film oxides", Thin Solid Films, 241 (1994) 198] beschreibt z. B. die Deposition von TiO₂. Eine den Metalloxidfilm überdeckende Beschichtung variierender Zusammensetzung kann hergestellt werden, indem zunnächst TEOS in die Vakuum-Kammer eingelassen wird und mit einem Ionenstrahl (Argon 5 keV) und der Spaltblende ein Ende des Metalloxidfilms beschichtet wird. Während dann die Spaltblende über den Metalloxidfilm bewegt wird, wird das TEOS in der Kammer in immer stärkerem Maße durch Titanpropylat [Ti(CH₃CH₂O)₄] ersetzt, was zu der Bildung eines Mischoxids führt, bis am anderen Ende des Metalloxidfilms reines TiO₂ als Beschichtung entsteht.

## Patentansprüche

1. Gassensor für reduzierende oder oxidierende Gase
a) mit einem Substrat (1), auf dem mehrere dünne gassensitive Felder bestehend aus einem halbleitenden Metalloxid-Dünnfilm angebracht sind, die
b) jeweils mit zwei Elektroden verbunden sind und
c) durch jeweils verschiedene Dotierung und/oder Beschichtung in Kontakt mit den reduzierenden oder oxidierenden Gasen unterschiedliche Leitfähigkeitsänderungen aufweisen;
dadurch gekennzeichnet, daß
d) die Felder aus einer einzigen, durchgehenden Schicht (3) bestehen,
e) die durchgehende Schicht durch zwei bandförmige äußere Elektroden (4) begrenzt wird,
f) zwischen den äußeren Elektroden (4) bandförmige innere Elektroden (4) angeordnet sind, durch die die durchgehende Schicht in die Felder aufgeteilt ist.

2. Gassensor nach Anspruch 1, dadurch gekennzeichnet, daß eine Beschichtung (5) auf der Schicht (3) vorgesehen ist, deren Durchlässigkeit für reduzierende oder oxidierende Gase sich zwischen den beiden äußeren Elektroden (4) stetig verändert.

3. Gassensor nach Anspruch 1, dadurch gekennzeichnet, daß eine Beschichtung (5) auf der Schicht (3) vorgesehen ist, die ein zu messendes Gas chemisch in ein reduzierendes oder oxidierendes Gas überführt.

4. Gassensor nach Anspruch 2, dadurch gekennzeichnet, daß die Beschichtung in einer zur durchgehenden Schicht (3) und zu den Elektroden (4) senkrechten Ebene einen keilförmigen Querschnitt aufweist.

5. Gassensor nach Anspruch 2, dadurch gekennzeichnet, daß die Durchlässigkeit für reduzierende oder oxidierende Gase durch stetige Veränderungen in der chemischen Zusammensetzung der Beschichtung (5) erzielt ist.

6. Gassensor nach Anspruch 1, dadurch gekennzeichnet, daß die Schicht (3) aus dem Metalloxid-Dünnfilm eine Dotierung aufweist, deren Zusammensetzung sich zwischen den beiden äußeren Elektroden (4) stetig ändert.

7. Gassensor nach einem der Ansprüche 1 bis 6, dadurch gekennzeichnet, daß die Schicht (3) auf einer Seite eines plattenförmigen Substrats (1) aufgebracht ist, und daß die andere Seite des Substrats (1) eine Heizung (2) mit mäanderförmig angelegten Wicklungen trägt, deren Fläche einem oder mehreren Feldern entspricht, wobei die Heizung (2) den entsprechenden Feldern gegenüberliegt.

8. Gassensor nach einem der Ansprüche 1 bis 7 mit einer Vorrichtung zur Bestimmung der elektrischen Leitfähigkeit der Felder, an die die Elektroden eines Feldes angeschlossen sind.

## Claims

1. Gas sensor for detecting reducing or oxidising gases,
a) having a substrate (1), on which a plurality of thin, gas-sensitive sections are situated, which sections are formed from a thin semiconductive metallic oxide film,
b) each section being connected to two electrodes and
c) having variable changes in conductivity because of a different doping and/or coating when in contact with the reducing or oxidising gases;
characterised in that
d) the sections comprise a single, continuous layer (3),
e) the continuous layer is defined by two strip-like outer electrodes (4), and
f) strip-like inner electrodes (4) are disposed between the outer electrodes (4) and divide the continuous layer into the sections.

2. Gas sensor according to claim 1, characterised in that a coating (5) is provided on the layer (3) and has a permeability to reducing or oxidising gases which constantly changes between the two outer electrodes (4).

3. Gas sensor according to claim 1, characterised in that a coating (5) is provided on the layer (3) and chemically converts a gas to be measured into a reducing or oxidising gas.

4. Gas sensor according to claim 2, characterised in that the coating has a wedge-shaped cross-section in a plane perpendicular to the continuous layer (3) or the electrodes (4).

5. Gas sensor according to claim 2, characterised in that the permeability to reducing or oxidising gases is achieved by constant changes in the chemical composition of the coating (5).

6. Gas sensor according to claim 1, characterised in that the layer (3), formed from the thin metallic oxide film, has a doping with a composition which varies constantly between the two outer electrodes (4).

7. Gas sensor according to one of claims 1 to 6, characterised in that the layer (3) is aplied to one side of a plate-like substrate (1), and in that the other side of the substrate (1) is provided with a heating means (2) having windings arranged in a serpentine-like manner, the area of said windings corresponding to one or more sections, and the heating means (2) being situated opposite the corresponding sections.

8. Gas sensor according to one of claims 1 to 7, having a means for determining the electrical conductivity of the sections with which the electrodes of a section communicate.

## Revendications

1. Détecteur de gaz pour des gaz réducteurs et oxydants
a) avec un substrat (1), sur lequel sont appliqués plusieurs champs minces sensitifs aux gaz se composant d'une couche mince d'oxyde métallique semi-conductrice, qui
b) sont reliées par deux électrodes et comportent
c) par dopage respectivement différent et/ou enduction en contact avec les gaz réducteurs ou oxydants des variations de conductibilité différentes,
caractérisé en ce que
d) les champs sont composés d'une unique couche continue (3),
e) la couche continue est limitée par deux électrodes (4) extérieures en forme de ruban,
f) entre les électrodes extérieures (4) sont disposées des électrodes (4) intérieures en forme de ruban, par lesquelles la couche continue est partagée en champs.

2. Détecteur de gaz selon la revendication 1,
caractérisé en ce qu'
une enduction (5) sur la couche (3) est prévue, dont la perméabilité pour des gaz réducteurs ou oxydants se modifie constamment entre les deux électrodes extérieures (4).

3. Détecteur de gaz selon la revendication 1,
caractérisé en ce qu'
une enduction (5) est prévue sur la couche (3), qui transforme un gaz à mesurer chimiquement en un gaz réducteur ou oxydant.

4. Détecteur de gaz selon la revendication 2,
caractérisé en ce que
l'enduction comporte dans un plan perpendiculaire par rapport à la couche continue (3) et aux électrodes (4) une section transversale en coin.

5. Détecteur de gaz selon la revendication 2,
caractérisé en ce que
la perméabilité pour des gaz réducteurs ou oxydants est obtenue par des modifications constantes de la composition chimique de l'enduction (5).

6. Détecteur de gaz selon la revendication 1,
caractérisé en ce que
la couche (3) hors de la couche mince d'oxyde métallique comporte un dopage dont la composition se modifie constamment entre les deux électrodes extrêmes (4).

7. Détecteur de gaz selon une des revendications 1 à 6,
caractérisé en ce que
la couche (3) est appliquée sur une face d'un substrat (1) en forme de plaque et l'autre face du substrat (1) porte un chauffage (2) avec des enroulements disposés en forme de méandres, dont la surface correspond à un ou plusieurs champs, le chauffage faisant face aux champs correspondants.

8. Détecteur de gaz selon une des revendications 1 à 7, avec un dispositif pour déterminer la conductibilité électrique des champs auxquels sont raccordées les électrodes d'un champ.
